# EUROPEAN PATENT APPLICATION

(11) **EP 1 872 744 A1**
(43) Date of publication of application: **02.01.2008**
(21) Application number: 06124618.7
(22) Date of filing: 23.11.2006
(51) Int. Cl.: A61F 2/36

(54) **Hip joint prosthesis**

(30) Priority: 29.06.2006 TR 200603325
(71) Applicant: HAVITCIOGLU, Hasan, 35315 Izmir (TR)
(72) Inventor: HAVITCIOGLU, Hasan, 35315 Izmir (TR)
(74) Representative: Iskender, Ibrahim

(57) **Abstract**

The invention is the hip joint prosthesis, which has a spherical bone head (1) to form a femur head corresponding to acetabular component and a body advancing after said head, and also at least one suspension element (5) located on said body extension and damping the tensions formed in various directions on the body extension.

## Description

### Technical Field

The invention relates to the hip joint prosthesis.

The invention relates particularly to the hip joint prosthesis that are used in fracture fixation for the hipbone fractures, in the repair of hip joint and deformities thereof and in various diseases where it is necessary to replace the joint with the prosthesis, according to the preamble of claim 1.

### Prior Art

There exist prosthesis indications for the hip joint, due to various reasons. Up to date, many studies have been carried out in vivo and in vitro about the practice and problems of a great variety of prosthesis and hip joint replacements. In historical development, it is mentioned in the publications that the first hip prosthesis was applied by John Rheu Barton on a hip anchylosed by intertrochanteric osteotomy and that some studies were carried out in the first half of the 1900's and until the 1960's. A significant development was achieved in the hip prosthesis upon the publication of the results of the total hip prosthesis, which was initiated in 1960's by Chanley and was completed in 1971, by the cement fixation in the femur component with the help of the polyethylene vessel causing a low friction in the acetabular component.

Owing to Chanley and his colleagues' use of the polyethylene vessel to enable a low friction in the acetabular component and also their fixation (adherence) of the prosthesis to the bone by means of the cement formed as a result of the polymerization following the mixing of polymethylmetacrylate and monomer, thus permitting a minimal possibility of micro movement, the bone ingrow (allowing the bone tissue ingress to the porous coated surface on the prosthesis) was achieved.

However, the problems with the hip prosthesis persist despite many studies and many clinical applications conducted. Such problems may be summarized as the biological problems (infection, soft tissue, bone ingrow, etc.), cement-related problems (polymethyl metacrylate) and prosthesis-related problems (mechanical). In literature, the relationships between the bone and the cement and the cement and the prosthesis have also been investigated. Non-cemented prosthesis has also been developed. The problems that exist with the non-cemented prosthesis and their long term effects are also available in the literature. Many hip prosthesis have been analyzed and developed. There also many studies available about the problems with acetabulum. The problems with the prosthesis may be categorized as the lubrification-related problems, polyethylene-related problems, material-related problems and loosening problems.

Today high molecular polyethylene is used in the acetabular component, for hip prosthesis. Many studies have been made especially about the high molecular polyethylene, for a minimal friction between the femur head of the prosthesis and the acetabulum and for a well lubrification. Also regarding the modular femur head, many studies have been made on the topics such as the head diameter and the ceramic head. Moreover, many analysis have been performed concerning the material used in the prosthesis. It was demonstrated that for the bone ingrow, no excessive micro movement must occur between the prosthesis-bone and between the cement-bone (especially during the early stages). Many studies have been carried out regarding the tension and stress distribution in the hip zone. In some biomechanical and clinical studies, the investigators showed that a mechanically imbalanced load distribution lead to cortical bone reduction and bone loss in proximal femur, in cases where the contact between the prosthesis and the bone is not appropriate. The complete proximal and distal filling of the femur canal by the prosthesis stem was shown to comprise an important factor in reducing the micro movement. Ninimaki et al., planned to increase the stem stability by using the prosthesis with isoelastic stems, but they observed excessive movement and consequently, osteolysis in the femur medial, as a result of the stem being elastic.

The mechanical reasons for the problem of loosening in the prosthesis basically include the inability for the anatomical hip model to fulfill its functions to an adequate extent. It is known that the flexibility module for the bone tissue is about 17.300 Mpa (cortical 20.000, cancellous 1000-5000 Mpa); while it is 200.000 Mpa for cobalt-chrome, 22.500 Mpa for carbon fiber and 20.000 Mpa for cement 1000-5000 Mpa, these being the prosthesis materials in use. It is observed that there is a great difference between the prosthesis material used and the bone tissue. In the isoelastic prosthesis used in the literature, the material with low flexibility module was employed in the isoelastic prosthesis. However, the flexibility module is higher for the cortical bone. Regarding the material currently in use in the prosthesis, we eventually use a material with a very high elasticity module, despite the use of very diverse metals. Niinimaki et al., used prosthesis with isoelastic stems, but they observed excessive movement in the femur proximal and consequently, osteolysis. Unfortunately, there is not any material available to take on the role of the joint cartilage playing a very important role in the hip joint or any component to perform this function. However, it is known that there occur changes in the load distribution and the stress shielding in the hip joint at various phases of walking and especially when descending and ascending the stair. In such phases, the load accumulation in particular may take place. Such accumulation is a negative factor for the bone ingrow. Today the ideal hip prosthesis has not yet been developed. In an ideal hip prosthesis, there must be a minimal friction, a well lubrification and an ideal stress shielding.

### Object of the Invention

Based on the known state of the art, the object of the invention is to enable a longer use and better load distributions between loosening (osteolysis), bone-cement, cement-bone for the prosthesis, which is provided an elastohydrodynamic characteristic owing to an elastic functional application in the hip prosthesis, such as the cartilage.

Another object of the invention is to ensure a better balanced load imparted on the prosthesis body and thus to obtain a better balanced load distribution between the prosthesis cement and the bone, hence ensuring a longer time for the staying of the prosthesis in human bone joint, by making the neck section of a prosthesis capable of load absorption by virtue of an improved hip joint prosthesis neck used in repair and restoration of the hip joint.

To achieve the said objectives, the hip joint prosthesis has been developed, which comprises a spherical bone head arranged in a way to form the femur head corresponding to the acetabular component and a body extending after said head.

According to a preferred embodiment of the invention, said hip joint prosthesis comprises at least one suspension element located on said body extension and damping the tensions formed in various directions on the body extension.

According to a preferred embodiment of the invention, said suspension element is provided with at least one housing formed on a section of said body extension, a projecting part formed on another section of the body extension and corresponding to said housing and at least one shock absorber element located between said housing and the projecting part.

According to a preferred embodiment of the invention, said shock absorber element is a spring. According to another preferred embodiment of the invention, said shock absorber element is silicone.

To achieve the said objectives, a preferred embodiment of the invention comprises at least one upper and at least one lower body part to constitute a shock absorbing structure, the housing formed on said upper and lower body parts and the projecting part fit for said housing and at least one shock absorber element connected between said housing and the corresponding projecting part from one end of each.

### Description of the Drawings

Figure-1 is the frontal section of an illustrative application of the invention, where the spring is used as the shock absorber.
Figure-2 is the frontal section of an illustrative application of the invention, where the silicone is used as the shock absorber.

### Reference Numbers

1. Spherical section
2. Conical section
3. Body upper extension
   3.1 Pressure cylinder
   3.2 Centering housing
4. Body lower extension
   4.1 Centering cylinder
   4.2 Flexible material housing
   4.3 Step wall folding
5. Flexible material

### Detailed Description of the Invention

The applications of the invention provided in Figure-1 and Figure-2 show the illustrative application of the hip joint prosthesis using a spring and silicone as the flexible material (5), respectively.

The prosthesis comprises a spherical section (1) which performs the function of femur head inside the acetabular section. The conical section (2) at the end of the cylindrical prosthesis upper body extension (3) fits into said spherical section (1). The lower end of the prosthesis body upper extension (3) advances in the form of a hollow cylinder narrowed by providing steps. The thickness of said formation establishes the pressure cylinder (3.1), while the space inside it forms the centering housing (3.2).

As in the upper extension (3), the lower body extension (4) also advances in the form of a cylinder with equal diameter and fits in the femur. The upper end of the lower extension (4) has a shape suitable for comprising a counterpart of for the lower and of the upper extension (3). In the middle, the flexible material housing (4.2) is provided with a space to house the pressure cylinder (3.1) in such a way to form the narrow cylindrical stepped core having a diameter suitable for ingress to the housing (3.2). The flexible material (5) like the spring or the silicone is located on the base of said housing (4.2) and the top surface of the pressure cylinder (3.1) contacts on the same. In this way, any tension imparted on the hip joint through the spherical section (1) will be absorbed by the flexible material (5) serving as a cartilage. This condition is provided by the connection between the lower and upper body extensions (3, 4) which constitute cylinders arranged one within the other. In order to limit the stretching distance, the step wall folding (4.3) is provided at a location where it can contact the starting step wall of the pressure cylinder (3.1). Furthermore, in order to ensure a linear advancement, the centering cylinder (4.1) forms a guide inside the centering housing (3.2) inside the pressure cylinder (3.1).

The invention may not be limited to the illustrative embodiments provided in this section. The alternative embodiments that may be performed by the persons skilled in the art based on the basic principles within the protective scope as defined in the claims will mean the violation of the invention.

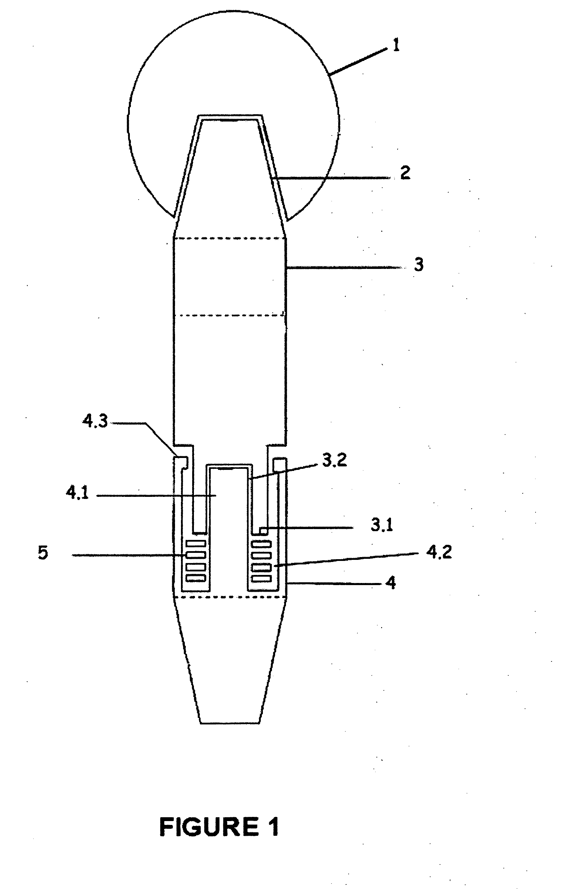

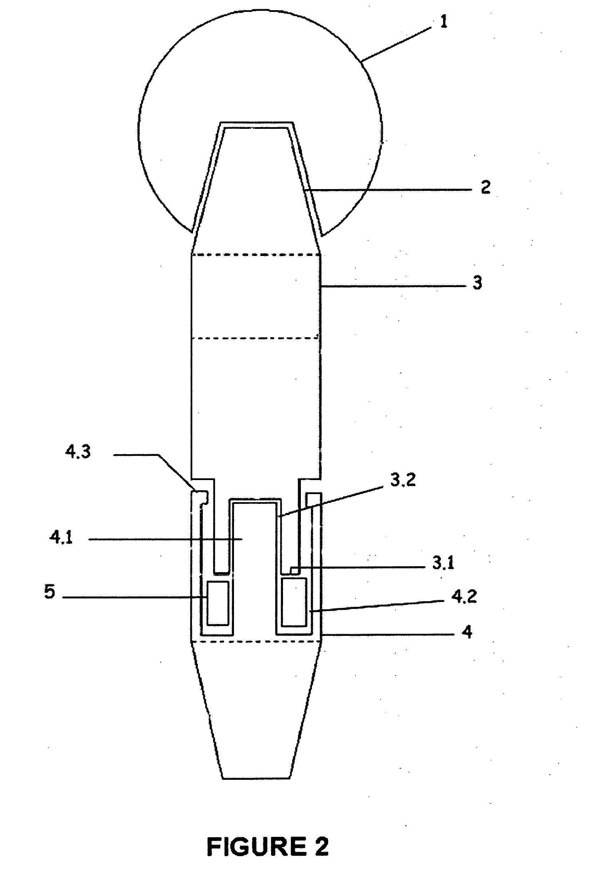

## Claims

1. The invention is a hip joint prosthesis having a spherical bone head (1) to form a femur head corresponding to acetabular component and a body advancing after said head **characterized in that** it comprises
- at least one suspension element (5) located on said body extension and damping the tensions formed in various directions on the body extension.

2. A hip joint prosthesis according to claim 1 **characterized in that** in order to provide said suspension element, it comprises
- at least one housing (4.2) formed on a part (3, 4) of said body extension,
- a projecting part (3.1) formed on another part (4, 3) of the body extension (3, 4) and corresponding to said housing (4.2) and
- at least one shock absorber element (5) located between said housing (4.2) and the projecting part (3.1).

3. A hip joint prosthesis according to claim 2 **characterized in that** said shock absorber element (5) is a spring.

4. A hip joint prosthesis according to claim 2 **characterized in that** said shock absorber element (5) is silicone.

5. The invention is a hip joint prosthesis having a spherical bone head (1) to form a femur head corresponding to acetabular component and a body advancing after said head (1) **characterized in that** it comprises
- at least one upper and at least one lower body part (3, 4) to form a shock absorbing structure,
- the housing (4.2) formed mutually on said upper and lower body parts (3, 4) and the projecting part (3.1) that fits the same,
- at least one shock absorber element (5) connected between said housing (4.2) and the corresponding projecting part (3.1) from one end of each.
